# Europäisches Patentamt
## European Patent Office
### Office européen des brevets

(11) Veröffentlichungsnummer: **0 031 826**
**B1**

(12) # EUROPÄISCHE PATENTSCHRIFT

(45) Veröffentlichungstag der Patentschrift:
**30.05.84**

(51) Int. Cl.³: **G 01 N 33/02, G 01 N 21/78**

(21) Anmeldenummer: **80901233.9**

(22) Anmeldetag: **11.07.80**

(86) Internationale Anmeldenummer:
**PCT/CH 80/00085**

(87) Internationale Veröffentlichungsnummer:
**WO 81/00303 (05.02.81 Gazette 81/4)**

(54) **VERFAHREN ZUR ÜBERWACHUNG DER ZEIT/TEMPERATURGESCHICHTE EINES TIEFGEKÜHLTEN GUTES, INDIKATOR ZUR AUSFÜHRUNG DES VERFAHRENS UND VERWENDUNG DES VERFAHRENS.**

(30) Priorität: **13.07.79 CH 6535/79**

(43) Veröffentlichungstag der Anmeldung:
**15.07.81 Patentblatt 81/28**

(45) Bekanntmachung des Hinweises auf die Patenterteilung:
**30.05.84 Patentblatt 84/22**

(84) Benannte Vertragsstaaten:
**DE FR GB SE**

(56) Entgegenhaltungen:
**CH - A - 415 107**
**US - A - 2 560 537**
**US - A - 4 042 336**

(73) Patentinhaber: **ALLMENDINGER, Thomas,
Bruggwiesenstrasse 7, CH-8152 Glattbrugg (CH)**

(72) Erfinder: **ALLMENDINGER, Thomas,
Bruggwiesenstrasse 7, CH-8152 Glattbrugg (CH)**

(74) Vertreter: **Troesch, Hans Alfred, Dr. Ing. et al,
Walchestrasse 19, CH-8035 Zürich (CH)**

ACTORUM AG

**Beschreibung**

Die vorliegende Erfindung betrifft einen Indikator zur Überwachung der Zeit/Temperaturgeschichte eines tiefgekühlten Gutes im Sinne eines Zeitintegrals seines Temperaturverlaufes, wobei der Diffusionsweg von Wasser auf einer Diffusionsstrecke erfasst wird, bestehend aus einem als Diffusionsstrecke dienenden, saugfähigen Zellulosekörper, der von einer feuchtigkeitsdichten Umhüllung umschlossen ist, wobei der Zellulosekörper mit einer Substanz bzw. einem Substanzgemisch versehen ist und gegebenenfalls einen Säure/Base-Indikator und/oder eine Zeitskala aufweist sowie Verfahren zur Überwachung der Zeit/Temperaturgeschichte eines tiefgekühlten Gutes im Sinne eines Zeitintegrals seines Temperaturverlaufes, wobei der Diffusionsweg von Wasser auf einer Diffusionsstrecke erfasst wird, mit einem derartigen Indikator und weiter eine Verwendung des Indikators zur Überwachung der Zeit/Temperaturgeschichte eines tiefgekühlten Gutes im genannten Sinne, wobei der Diffusionsweg von Wasser auf einer Diffusionsstrecke erfasst wird.

Es besteht schon lange das Bedürfnis, tiefgekühlte Lebensmittel zu überwachen. Hierzu wird auf die US-A-1 535 536 aus dem Jahre 1925, zur Überwachung der Lagertemperatur von Eiscreme, hingewiesen. Dieser Veröffentlichung folgten eine Reihe weiterer, worin man sich mit der Frage befasste, wie man die Unsicherheit in der Lagertemperatur von Tiefkühlprodukten mittels eines einfachen und billigen Indikators erfassen könne. Hierzu wird auf die Publikation von H.M. Schön und C.H. Byrne in «FOOD TECHNOLOGY», Oktober 1972 hingewiesen, in welcher derartige Veröffentlichungen übersichtsmässig erwähnt sind. Die meisten herkömmlichen Indikatoren beruhen auf dem Prinzip, dass eine Grenztemperatur an den Tiefkühlprodukten überwacht wird, in dem ein wenn auch nur kurzzeitiges Überschreiten dieser Temperatur entweder sofort oder nach einiger Verzögerung, in der Grössenordnung von Stunden, aufgezeigt wird. Solche Indikatoren sind unter dem Begriff «Defrost Indicators» bekannt. Sie funktionieren meistens nach dem Prinzip, dass eine bei der erforderlichen Lagertemperatur gefrorene Flüssigkeit nach Überschreiten der Grenztemperatur oberhalb —18°C schmilzt und von einem Docht oder Papierstreifen aufgesogen wird, dann zu einem Anzeigefeld fliesst, wo sie einen Farbumschlag bewirkt. Diesbezüglich sei beispielsweise auf das französische Patent FR-A-1 548 424 oder die US-A-2 951 764 verwiesen.

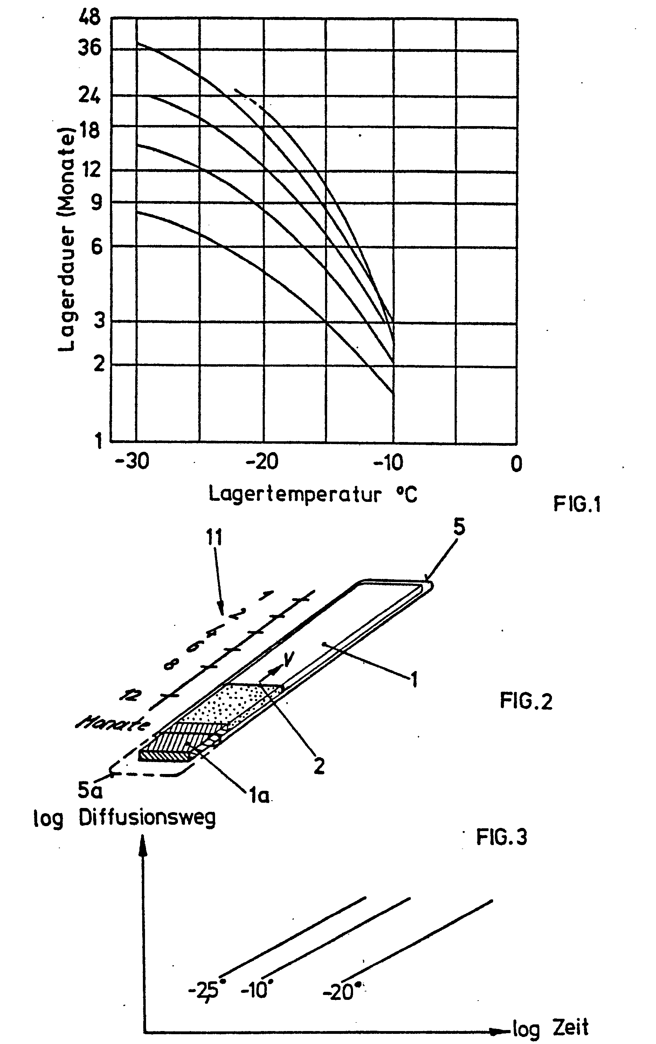

In neuerer Zeit hat man die Haltbarkeit von Tiefkühlprodukten jedoch genauer untersucht und gefunden, dass es keine bestimmten kritischen Temperaturen gibt, sondern dass die zulässige Lagerzeit von Tiefkühlprodukten mit steigender Temperatur stetig abnimmt, und zwar erfolgt die Qualitätsabnahme ungefähr logarithmisch. Mittlerweile ist es üblich geworden, mittels sog. Haltbarkeitskurven graphisch darzustellen, nach welchen Zeiten, in Abhängigkeit der Temperatur, eine noch zulässige Qualitätslimite erreicht wird. In Fig. 1 sind einige Haltbarkeitskurven für verschiedene Produktarten dargestellt, die der Veröffentlichung «Recommandations

pour la préparation et la distribution des aliments congelés, 2e Edition, 1972, Pages 118/119» des Institut International du Froid, Paris, entnommen sind.

Diese Haltbarkeitskurven sind für verschiedene Produktklassen unterschiedlich, wobei sowohl die maximal zulässige Lagerdauer bei —20°C als auch die Temperaturabhängigkeit der Lagerfähigkeit unterschiedlich sein können. Die Häufigkeit der Temperaturschwankungen spielt ausser bei Eiscreme normalerweise eine zu vernachlässigende Rolle. In neuerer Zeit ist es üblich geworden, für die Temperaturabhängigkeit der Haltbarkeit von Tiefkühlprodukten den sog. $Q_{10}$-Wert zu verwenden. Darunter versteht man den Quotienten, der sich daraus ergibt, dass man die Haltbarkeitsdauer eines bestimmten Produktes bei einer bestimmten Temperatur durch die Haltbarkeitsdauer dividiert, welche dieses Produkt bei einer um 10°C höheren Temperatur aufweist. Weil aber dieser $Q_{10}$-Wert temperaturabhängig ist, also beispielsweise im Bereich zwischen —2,5°C und —12,5°C einen anderen Wert als im Bereich zwischen —10°C und —20°C aufweist, soll hier zur Charakterisierung der Temperaturabhängigkeit anstelle des erwähnten $Q_{10}$-Wertes der $Q_{-20°/-2,5°C}$-Wert eingeführt werden, welcher den für Tiefkühlprodukte interessanten Lagerbereich zwischen —20°C und —2,5°C, knapp vor dem Auftauen, umfasst.

Gemäss einer groben Einteilung betragen die $Q_{-20°/-2,5°C}$-Werte für Gemüse und Früchte ca. 45, für Fleisch und Fisch ca. 20. Andere Produktklassen liegen zwischen diesen Extremfällen.

Bei in neuerer Zeit bekanntgewordenen Indikatoren wird das Erreichen einer für ein Tiefkühlprodukt charakteristischen Qualitätslimite in Abhängigkeit von der Temperatur so angezeigt, wie es der Haltbarkeitskurve dieses Produktes entspricht. Dies wird dadurch erreicht, dass in einem Streifen eine Flüssigkeit empordiffundiert, und zwar zum so rascher, je wärmer die Umgebung des Streifens ist. Wird einem Tiefkühlprodukt ein solches Streifchen kurz nach dem Einfrieren einverleibt, so erfasst dieses sämtliche im weiteren Verlauf eintretenden Veränderungen, indem die Flüssigkeit rascher zu fliessen beginnt, wenn es einmal wärmer werden sollte, dann wieder langsamer, wenn die Temperatur sinkt. Da der Vorgang irreversibel ist, werden alle Qualitätsverminderungen infolge Temperaturanstiegs aufintegriert. Derartige Indikatoren sind unter dem Begriff «Time Temperature Integrator» bekannt. Diesbezüglich sei auf folgende Publikationen verwiesen:

— A. Kramer and J.W. Farquhar: Testing of Time-Temperature indicating and Defrost Devices, Food Technology, Feb. 1976, p. 50-56,

— C.H. Byrne: Temperature Indicators — the State of the Art, Food Technology, June 1976, p. 66-68,

— H. Schubert: Indikatoren zur Kontrolle der Zeit-Temperatur-Belastung von tiefgefrorenen Lebensmitteln, Zeitschrift für Lebensmittel-Technologie und -Verfahrenstechnik, *31* (1980) 3, S. 137-142.

(Dieser Artikel ist allerdings erst nach unserer Erstanmeldung in der Schweiz erschienen.)

Bei diesen neueren Time/Temperature-Integratoren diffundiert normalerweise eine Flüssigkeit mit temperaturabhängiger Viskosität bzw. Geschwindigkeit aus einem Vorrat entlang eines saugfähigen Streifens (vgl. z.B. US-A-3 954 011). Im US-A-3 946 611 von Larsson ist dagegen als Vorrat ein gaserzeugender Stoff vorgesehen. Das erzeugte Gas wird dann entlang der Diffusionsstrecke von einem geeigneten Stoff absorbiert. Die Möglichkeit, dabei Wasserdampf als Gas zu verwenden, wird zwar erwähnt, jedoch wird nicht auf die für diesen Fall zur Absorption in Betracht kommenden Substanzen eingegangen. Die Art dieser Substanzen ist nämlich von grosser Bedeutung, bestimmen sie doch im wesentlichen den Q-Wert der Diffusion. Mit den gemeinhin als hygroskopisch bekannten Substanzen, wie z.B. Kalziumchlorid, Magnesiumchlorid oder Kobaltchlorid lassen sich jedoch nur $Q_{-20°/-2,5°C}$-Werte bis max. 10 erreichen, was weit unter dem erforderlichen Maximalwert von ca. 50 liegt.

Ausserdem sind bei der Wahl der zur Absorption dienenden Substanzen noch andere Kriterien, wie Deutlichkeit der Diffusionsfront, Toxizität, usw. zu berücksichtigen. Auf diese Fragen wird in der US-A-3 946 611 Larsson nicht eingegangen. Vielmehr geht aus den dort angeführten Beispielen hervor, dass als gaserzeugender Stoff vorzugsweise eine Säure oder eine Base und als gasadsorbierender Stoff umgekehrt eine Base oder eine Säure vorgesehen ist, d.h. dass der Absorptionsvorganng eine Säure/Base-Reaktion ist. Ausserdem sind Redox-Vorgänge sowie Komplexreaktionen für die Absorption/Indikation vorgesehen. Gemäss diesem Patent von Larsson ist eine semipermeable Membran zwischen Gasvorrat und Diffusionsstrecke vorgesehen, welche die Temperaturabhängigkeit des Diffusionsvorgangs steuern soll. Im Folgepatent US-A-4 042 336 von Larsson ist auf der Diffusionsstrecke zusätzlich eine chemische Substanz («quantifier») vorgesehen, welche die Temperaturabhängigkeit des Diffusionsvorgangs steuert. Die Steuerung erfolgt aufgrund einer Säure/Base-Reaktion.

Die vorliegende Erfindung bezweckt einen Indikator eingangs genannter Art zu schaffen, der mit besonders einfachen Mitteln und mit ungiftigen und billigen Substanzen realisierbar ist.

Er zeichnet sich dadurch aus, dass die Umhüllung eine Kontaktierungsöffnung für einen Wasser- bzw. Eisvorrat aufweist und der Zellulosekörper mit einer wasserlöslichen, hydratbildenden Substanz bzw. einem Substanzgemisch versehen ist.

Durch das Vorsehen eines hygroskopischen Stoffes auf der Diffusionsstrecke wird es möglich, als Flüssigkeitsvorrat nicht eine wässrige Lösung mit tiefliegendem Schmelzpunkt herbeiziehen zu müssen, sondern reines Eis verwenden zu können, über welchem im abgeschlossenen Raum 100%ige Luftfeuchtigkeit herrscht und welches dadurch als Wasserlieferant für die mit dem hygroskopischen Stoff versehene Diffusionsstrecke dienen kann.

Eines der wesentlichen Merkmale dieser Erfindung ist es nun aber, nicht oder nicht unbedingt eine als gemeinhin bekannte hygroskopische (englisch: «deliquescent», an der Luft zerfliessend) Substanz, wie Kalziumchlorid, Natriumhydroxid, Phosphorpentoxid usw. zu verwenden, sondern die Erkenntnis auszunützen, dass bei 100%iger relativer Luftfeuchtigkeit, so wie sie über reinem Wasser bzw. Eis im abgeschlossenen Raum herrscht, jeder wasserlösliche Stoff innerhalb seines Löslichkeitsbereiches hygroskopisch wird, also Feuchtigkeit aufzunehmen vermag. Damit auf einer mit einem solchen Stoff versehenen Diffusionsstrecke, wie einem Zellulosekörper, eine deutliche Front sichtbar wird, sollte dieser Stoff allerdings ein Hydrat bilden.

Es hat sich gezeigt, dass die gemeinhin als stark hygroskopisch bekannten Substanzen, wie Kalziumchlorid, Natriumhydroxyd, Phosphorpentoxid usw. nur eine geringe Temperaturabhängigkeit der Diffusionsgeschwindigkeit von Wasser zur Folge haben, also lediglich die Erfassung sehr kleiner $Q_{-20°/-2,5°C}$-Werte erlauben, abgesehen davon, dass die meisten dieser Substanzen lebensmittelhygienisch nicht unbedenklich sind, in diesem Zusammenhang ein sehr wesentliches Kriterium.

Die in jedem Haushalt anzutreffenden Substanzen, Natriumchlorid, Zitronensäure und Zucker, ergeben dagegen einen deutlich sichtbaren, wenn auch nicht mit einem Farbumschlag verbundenenn Diffusionsverlauf hoher Temperaturabhängigkeit, so dass, z.B. mit einem Natriumchlorid/Zitronensäuregemisch im Gewichtsverhältnis 2:1, ein $Q_{-20°/-2,5°C}$-Wert von bis zu 50 erreichbar ist.

Es sei vermerkt, dass die Zitronensäure hier nicht in ester Linie wegen ihrer Säure-Eigenschaften, sondern wegen ihrer hygroskopischen Eigenschaften Verwendung findet. Unterhalb des kryohydratischen Punktes, der für Natriumchlorid ungefähr bei —22°C, bei Zitronensäure ungefähr bei —15°C liegt und bei Gemischen etwas tiefer, kommt der Absorptions- und Diffusionsvorgang zum Stillstand, weil dann die Substanzen nicht mehr wasserlöslich sind.

Während sich für die Überwachung der meisten Tiefkühlprodukte die Verwendung eines Zitronensäure/Natriumchloridgemisches als besonders geeignet erwiesen hat, empfiehlt sich für die Überwachung von Glace die Verwendung reiner Zitronensäure.

Bei einem solchen Indikator, bestehend aus einem als Diffusionsstrecke dienenden Zellulosekörper, der von einer feuchtigkeitsdichten Umhüllung 5, 15, 16 umschlossen ist, muss die Umhüllung 5, 15, 16 eine Kontaktierungsöffnung 1a, 21 für das Wasser aufweisen. Die feuchtigkeitsdichte Umhüllung ist deshalb vorgesehen, weil der Körper bis auf die Kontaktstelle keine Feuchtigkeit aufnehmen darf. Da keine semipermeable Folie die Diffusionsstrecke vom Wasservorrat trennt, wird erreicht, dass die Diffusionsgeschwindigkeit in dem Moment wesentlich grösser wird, in welchem der Wasservorrat schmilzt, also beim Überschreiten der 0°C-Grenze. Dieser Zusatzeffekt ginge bei Verwendung einer semipermeablen Membran praktisch verloren. Verwendet man als Wasser- bzw. Eisvorrat nicht reines Wasser, sondern beispielsweise Eiscreme, so hat dies weitere, für die Anzeige günstige Effekte zur Folge.

Je grösser nämlich der Anteil an gelösten Stoffen im Wasservorrat ist, um so rascher diffundiert die Flüssigkeitsfront auf der Diffusionsstrecke. Dieser Effekt korreliert positiv mit der Tatsache, dass die zuckerreiche Fruchtglace auch weniger lang haltbar ist als die zuckerärmere Rahmglace.

Bei der vorgeschlagenen Erfindung handelt es sich somit um eine Kombination zwischen einem Time/Temperature-Integrator und einem Defrost-Indikator.

Am einen, der Kontaktierungsöffnung gegenüberliegenden Ende des Zellulosekörpers wird zum Anzeigen eines vorgegebenen Diffusions-Grenzwertes ferner die Anbringung von Lackmuspapier als Säure/Base-Indikator vorgeschlagen, wobei in diesem Fall der Zellulosekörper mindestens mit Zitronensäure versehen sein muss. Diese ursprünglich feste Säure wird vom empordiffundierenden Wasser aufgelöst und zum Säure/Base-Farbindikator getragen. Der Farbumschlag an diesem Indikator zeigt dem Konsumenten somit das Erreichen einer bestimmten Qualitätslimite des kontrollierten Gutes an.

Der Indikator wird aktiviert durch Kontaktierung mit einem Wasser- bzw. Eisvorrat, wobei als Vorrat der Feuchtigkeitsgehalt des Gutes selbst oder ein zusätzlich vorgesehener Wasser- bzw. Eisvorrat dienen kann. Im einfachsten Fall, z.B. bei Fleisch, welches in einer Klarsicht-Schrumpffolie verpackt wird, oder bei Glace, kann der Feuchtigkeitsgehalt des zu überwachenden Produktes selber verwendet werden. In anderen Fällen muss in der Verpackung des Produktes eine kleine Vertiefung vorgesehen werden, in welcher etwas Wasser zudosiert werden kann. Im Gegensatz zum Vorsehen eines Wasservorrates am Indikator selbst wird dadurch auch sichergestellt, dass keine vorzeitige, unerwünschte Aktivierung des Indikators erfolgen kann.

Ist die Diffusionsstrecke mit einem Natriumchlorid/Zitronensäuregemisch versehen, so findet bei der Lagerung des unaktivierten Indikators allmählich ein vorzeitiger, unerwünschter Farbumschlag des Indikators, beispielsweise des Lackmuspapiers, von blau nach rot statt, weil sich aus obigem Gemisch etwas Chlorwasserstoff bilden kann, welcher aufgrund von Ionenwanderung zum Lackmuspapier diffundiert.

Um diesen vorzeitigen Farbumschlag zu verhindern, wird vorgeschlagen, dass der Körper, falls er auf der Diffusionsstrecke mit einem Natriumchlorid/Zitronensäuregemisch versehen ist, vom Säure/Base-Farbindikator durch ein Streckenstück mit einem Puffer, vorzugsweise mit sekundärem Ammoniumcitrat, getrennt wird. Die so gebildete Pufferstrecke absorbiert den unerwünschterweise entstehenden Chlorwasserstoff dank seiner basischen Eigenschaften.

Da es aus praktischen Gründen mitunter unumgänglich ist, den Indikator bereits mit dem Wasservorrat in Kontakt zu bringen, kurz bevor das Wasser gefroren ist, indem man beispielsweise in die dafür vorgesehene Vertiefung einer bereits tiefgefrorenen Packung zuerst Wasser zudosiert und unmittelbar darnach den Indikator in Form einer Selbstklebeetikette darüberklebt, wird vorgeschlagen, dass im Bereich der Kontaktierungsstelle für das Wasser am Körper eine zähflüssige Substanz, vorzugsweise ein Zucker, vorgesehen ist, welcher bei der Aktivierung des Indikators ein vorzeitiges Emporfliessen des Wassers verhindert.

Als Umhüllung des Zellulosekörpers kann entweder eine anhaftende transparente Kunststoffolie, beispielsweise eine Polychlortrifluoräthylen-Klebefolie, verwendet werden, oder insbesondere für Glace, eine Kunststoffausformung, vorzugsweise eine tiefgezogene Form aus transparentem PVC.

Die Länge des Zellulosekörpers in Diffusionsrichtung kann auf die maximale Haltbarkeit des zu überwachenden Gutes, vorzugsweise bei —20°C, abgestimmt werden, wobei mittels einer zusätzlich vorgesehenen, logarithmischen Zeitskala die Resthaltbarkeit des zu kontrollierenden Gutes abgelesen werden kann.

Die Temperaturabhängigkeit des Diffusionsverlaufs kann dadurch einfacherweise an die Temperaturabhängigkeit des zu überwachenden Gutes angepasst werden, dass das Mischungsverhältnis von Natriumchlorid und Zitronensäure der Temperaturabhängigkeit der Haltbarkeit des zu überwachenden Gutes angepasst wird.

Dank dieser Anpassungsmöglichkeiten kann der vorgeschlagene Indikator optimal zur Überwachung des Qualitäts- bzw. Lagerzustandes von tiefgekühlten Lebensmitteln im Temperaturbereich von —25°C bis +5°C herangezogen werden, indem einerseits eine Diffusionsfront entlang des Diffusionskörpers die Abnahme der Haltbarkeit des zu überwachenden Gutes mit Hilfe einer auf eine bestimmte Temperatur, vorzugsweise auf —20°C, bezogenen Zeitskala anzeigt und dass andererseits ein deutlicher Farbumschlag des Säure/Base-Indikators am Ende der Diffusionsstrecke das Erreichen eines vorgegebenen Diffusionsgrenzwertes und damit das Verfallsdatum des Gutes anzeigt.

### Kurze Beschreibung der Zeichnungen

Die Erfindung wird anschliessend beispielsweise anhand von Figuren erläutert.

Es zeigen:

Fig. 1 die Abhängigkeit der zulässigen Lagerdauer verschiedener Tiefkühlprodukte in Abhängigkeit ihrer Lagertemperatur,

Fig. 2 eine perspektivische Ansicht eines Indikators, ohne Farbumschlag,

Fig. 3 die qualitative Abhängigkeit des Diffusionsweges von der Zeit, an einem Integrator gemäss Fig. 2, mit der Temperatur als Parameter,

Fig. 4 eine Seitenansicht einer weiteren Ausführungsform des Indikators gemäss Fig. 2, mit Farbumschlag,

Fig. 5 eine prinzipielle Darstellung eines auf dem zu überwachenden Gut angebrachten Indikators,

Fig. 6 eine prinzipielle Darstellung eines an der Verpackung eines zu überwachenden Gutes aufgebrachten Indikators,

Fig. 7a einen in einer Kunststoffvertiefung eingebrachten Indikator für Glace,

Fig. 7b einen in einer Verpackungswand eingefügten Indikator für Glace.

Auf die in Fig. 1 dargestellten sog. Haltbarkeitskurven wurde in der Abhandlung des Standes der Technik Bezug genommen. Der in Fig. 2 dargestellte Indikator umfasst einen Papierstreifen 1. Dieser ist mit einer wasserlöslichen, hydratbildenden Substanz wie Natriumchlorid, Zitronensäure, einem Zucker oder einem Gemisch zweier oder mehrerer dieser Substanzen versehen. Mit 2 ist auf dem Indikator die mit der Geschwindigkeit v vordiffundierende wässrige Front dargestellt. Der Streifen 1 ist von einer feuchtigkeitsdichten Folie 5 umschlossen, vorzugsweise einer Chlortrifluoräthylen-Klebefolie. Diese Folie lässt eine Kontaktstelle 1a des Streifens, mindestens auf einer Seite, wie gestrichelt bei 5a dargestellt, frei. Somit ist die Kontaktstelle 1a so ausgebildet, dass sie direkt mit dem Wasservorrat am Gut, sei dies der Feuchtigkeitsgehalt des Gutes selbst, sei dies ein zusätzlich zugeführter Wasser- oder Eisvorrat am Gut oder dessen Verpackung kontaktierbar ist.

Um zu verhindern, dass bei der Kontaktierung der Kontaktstelle 1a mit Wasser dieses sofort hochzudiffundieren beginnt, was dann der Fall ist, wenn der Indikator unmittelbar vor dem Einfrieren dieses Wasservorrates angebracht wird, ist die Kontaktstelle 1a mit einer zähflüssigen Substanz, beispielsweise mit einem Zucker versehen, was durch die Schraffur bei 1a in Fig. 2 dargestellt ist.

Setzt man verschiedene, anhand der Fig. 2 beschriebene Indikatoren je unterschiedlichen Temperaturen aus, so werden die in Fig. 3 qualitativ aufgezeigten Diffusionswegabhängigkeiten von der Zeit gefunden, wobei sowohl der Diffusionsweg wie auch die Zeit logarithmisch abgetragen sind. Als Versuchstemperaturen wurden die Temperaturen —20°C, —10°C und —2,5°C gewählt. Im Bereich über 0°C ist die Diffusionsgeschwindigkeit noch wesentlich grösser, da die Kontaktstelle 1a in direktem Kontakt mit Wasser steht, weil das vorgängig kontaktierte Eis geschmolzen ist und dann kein Umweg des Wassers über die Dampfphase mehr nötig ist. Aus einem Diagramm, wie in Fig. 3 gezeigt, lässt sich ohne weiteres ablesen, wie lange die Diffusionsfront bei den einzelnen Temperaturen braucht, um eine bestimmte Wegstrecke zurückzulegen. Trägt man die so erhaltenen Diffusionszeiten für eine vorgegebene Wegstrecke gegen die Temperatur auf, so werden Kurven erhalten, entsprechend Haltbarkeitskurven von Tiefkühlprodukten. Da jedes einigermassen feuchte Tiefkühlprodukt angenähert einem Eisklotz gleichgestellt werden kann, ist die Abhängigkeit des Fliessvorganges von der Art des Tiefkühlproduktes praktisch unabhängig, sofern keine gelösten Substanzen wie Zucker, Kochsalz usw. im Produkt vorhanden sind. Anderseits ist jedoch die Lagerfähigkeit sehr wohl davon abhängig, so dass es nötig ist, für jede Tiefkühlproduktart einen entsprechend langen Körper, entsprechend dem Papierstreifen 1, vorzusehen, wobei durch Verminderung des Mischungsverhältnisses der aufgebrachten Substanzen, insbesondere von Natriumchlorid und Zitronensäure, die Temperaturabhängigkeit der Diffusionsgeschwindigkeit derjenigen der Haltbarkeit des zu überwachenden Gutes angepasst wird.

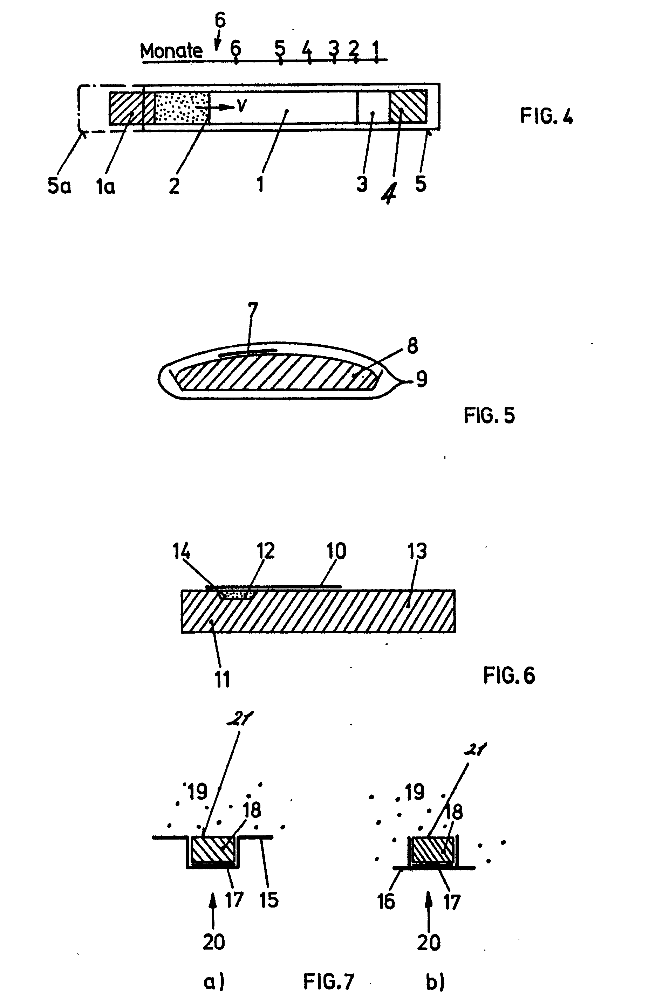

Der in Fig. 4 dargestellte Indikator weist nebst den Papierstreifen 1, der feuchtigkeitsdichten Folie 5 und der an der Kontaktierungsstelle 1a aufgebrachten zähflüssigen Substanz, vorzugsweise Zucker, an seinem der Kontaktierungsstelle 1a entgegengesetzten Ende einen Säure/Base-Indikator, vorzugsweise ein Lackmuspapier 4 auf. Auf dem Papierstreifen 1 ist dabei eine wasserlösliche Säure, vorzugsweise Zitronensäure, aufgebracht, die vom empordiffundierenden Wasser, mit der Front 2, gelöst und zum Säure/Base-Indikator 4 emporgetragen wird. Um nun zu verhindern, dass der Säure/Base-Indikator 4 vorzeitig, beispielsweise durch Bildung von Chlorwasserstoffgas und dessen Empordiffundieren zum Umschlag gebracht wird, ist auf einer Wegstrecke zwischen dem Säure/Base-Indikator 4 und der Kontaktstelle 1a eine Strecke 3 zwischengeschaltet, welche mittels eines Puffers, vorzugsweise mittels sekundärem Ammoniumcitrat, versehen ist. Diese Pufferstrecke absorbiert dank ihrer basischen Eigenschaften unerwünschten Chlorwasserstoff. Am Indikator ist weiter, wie mit 6 dargestellt, eine logarithmische Zeitskala vorgesehen, welche auf die Resthaltbarkeit des zu kontrollierenden Gutes bei einer bestimmten Temperatur, beispielsweise bei —20°C bezogen wird.

Der anhand der Fig. 2 und 4 dargestellte streifenförmige Indikator kommt insbesondere dort zur Anwendung, wo als wasserlösliche, hydratbildende Substanz ein Kochsalz/Zitronensäuregemisch verwendet wird. Dies ist bei allen Tiefkühlprodukten, ausser bei Glace, der Fall. Die Fliessstrecke ist hier relativ lang, und zwar in der Grössenordnung einiger Zentimeter.

Wie in Fig. 5 dargestellt, kann dieser streifenförmige Indikator entweder direkt auf das Tiefkühlprodukt 8 gelegt werden, sofern das Ganze anschliessend noch mit einer Kunststofffolie 9 verpackt wird, wie dies beispielsweise bei Fleisch der Fall ist, oder er kann in Form einer Selbstklebeetikette 10, wie in Fig. 6 dargestellt, auf eine Verpackung 11 geklebt werden, welche in einer Vertiefung 12 einen kleinen Wasservorrat 14 aufweist.

Der gemäss den Fig. 3 und 4 durch den Papierstreifen gebildete Zellulosekörper als Diffusionsstrecke kann in einer weiteren Ausführungsform zylinder- oder quaderförmig ausgebildet sein. Diese Ausbildungsform kommt insbesondere dann zur Anwendung, wenn als wasserlösliche, hydratbildende Substanz reine Zitronensäure verwendet wird, was bei der Überwachung von Glace der Fall ist. Die Fliessstrecke ist hier relativ kurz und zwar in der Grössenordnung einiger Millimeter, weil die Zitronensäure wegen ihrer relativ hohen Viskosität im flüssigen Zustand eine geringe Diffusionsgeschwindigkeit zur Folge hat. Bei —20°C und tiefer findet keine Reaktion statt. Dieser in den Fig. 7a und 7b dargestellte Indikator umfasst einen mit Zitronensäurelösung getränkten Filterkarton 18, Zylinder- oder Quaderform. Diese hier den Körper bildenden Pillen 18 sind in entsprechende Ausformungen aus Kunststoff, vorzugsweise aus PVC, eingelassen.

Gemäss Fig. 7a ist beispielsweise in der Verpackung der zu überwachenden Glace eine bezüglich des Gutes 19 nach aussen ragende Vertiefung vorgesehen, in welcher erst ein Säure/Base-Indikator 17, Lackmuspapier, und dann die mit Zitronensäure getränkte Pille 18 eingelegt wird. In Fig. 7b ist eine an der Verpackung der zu überwachenden Glace angebrachte Kunststoffhülse 16, beispielsweise aus PVC, dargestellt, welche in das Gut 19 einragt und in welche ebenfalls ein Säure/Base-Indikator 17, vorzugsweise Lackmuspapier, und anschliessend die mit Zitronensäure getränkte Pille eingelegt ist. In beiden in den Fig. 7a und 7b dargestellten Ausführungsformen ist die Kunststoffumhüllung mindestens an den Partien 20 klarsichtig, so dass der Farbumschlag des Indikators 17 von aussen beobachtet werden kann. Die Pille 18 kontaktiert das Gut 19, d.h. die zu überwachende Glace.

Die hier beschriebenen Indikatoren zur Überwachung der Zeit/Temperaturgeschichte eines tiefgekühlten Gutes eignen sich besonders für den Temperaturbereich von ca. —25°C bis ca. +5°C. Dabei kann mittels Variation der Körperlänge eine Anpassung an die maximale Haltbarkeit des Tiefkühlproduktes bei einer bestimmten Temperatur, vorzugsweise bei —20°C, und mittels Variation des Natriumchlorid/Zitronensäure-Verhältnisses, eine Anpassung an die Temperaturabhängigkeit der Haltbarkeit vorgenommen werden. Zur Eichung des Indikators bzw. zur Erfassung seiner Temperaturabhängigkeit kann ein Fliessdiagramm erstellt werden, indem der Logarithmus der Fliesszeit gegen den Logarithmus des Diffusionsweges aufgetragen wird, wie dies in Fig. 3 ersichtlich ist. Die dabei erhaltenen Geraden lassen eine gute Extrapolation zu.

Der Indikator gemäss den Fig. 2 und 4 wird so hergestellt, dass man zuerst einen Papierstreifen mit einer Kochsalz/Zitronensäurelösung tränkt und in der Hitze trocknet. Dann wird das eine Ende kurz in eine konzentrierte Zuckerlösung getaucht und erneut getrocknet. Das Pufferstück 3 wird analog hergestellt. Das Ganze wird zusammen mit einem Stück Lackmuspapier 4 in der richtigen Reihenfolge zwischen zwei Streifen einer gutanhaftenden transparenten Kunststoffolie, vorzugsweise einer Chlortrifluoräthylen-Klebefolie, gelegt, wobei ein Teil 5a dieser Folie etwas länger sein kann und die Kontaktstelle etwas überlappt, um so gegen Feuchtigkeitsverlust nach aussen hin zu schützen. Die Ausführungsform gemäss den Fig. 7a und 7b wird so hergestellt, dass man aus einem mit Zitronensäurelösung getränkten und anschliessend getrockneten Filterkarton eine oder mehrere Pillen ausstanzt und diese in die zylindrische oder quaderförmige Kunststoffausnehmung einschichtet, wobei zuerst der Lackmusindikator eingelassen wird.

Das beschriebene Verfahren und der Indikator zu dessen Ausführung ergibt eine zuverlässige, unaufwendige und damit billige Möglichkeit, Tiefgefrierprodukte bezüglich ihrer Haltbarkeit zu überwachen, was insbesondere bei Stromausfällen in Tiefgefrieranlagen von zunehmender Bedeutung sein kann.

**Patentansprüche**

1. Indikator zur Überwachung der Zeit/Temperaturgeschichte eines tiefgekühlten Gutes im Sinne eines Zeitintegrals seines Temperaturverlaufes, wobei der Diffusionsweg von Wasser auf einer Diffusionsstrecke erfasst wird, bestehend aus einem als Diffusionsstrecke dienenden, saugfähigen Zellulosekörper (1, 18), der von einer feuchtigkeitsdichten Umhüllung (5, 15, 16) umschlossen ist, wobei der Zellulosekörper mit einer Substanz bzw. einem Substanzgemisch versehen ist und gegebenenfalls einen Säure/Base-Indikator und/oder eine Zeitskala aufweist, dadurch gekennzeichnet, dass die Umhüllung (5, 15, 16) eine Kontaktierungsöffnung (1a, 21) für einen Wasser- bzw. Eisvorrat aufweist und der Zellulosekörper (1, 18) mit einer wasserlöslichen, hydratbildenden Substanz bzw. einem Substanzgemisch versehen ist.

2. Indikator nach Anspruch 1, dadurch gekennzeichnet, dass die wasserlösliche, hydratbildende Substanz Natriumchlorid, Zitronensäure, ein Zucker, vorzugsweise Natriumchlorid und/oder Zitronensäure oder ein Gemisch zweier oder mehrerer dieser Substanzen ist.

3. Indikator nach Anspruch 1, dadurch gekennzeichnet, dass am Ende des Zellulosekörpers (1, 18) ein Säure/Base-Indikator, vorzugsweise Lackmuspapier (4, 17), zum Anzeigen eines vorgegebenen Diffusionsgrenzwertes angebracht ist und dass die Substanz eine feste Säure oder Base enthält.

4. Indikator nach Anspruch 3, dadurch gekennzeichnet, dass der Zellulosekörper (1, 18) mit einem Natriumchlorid-Zitronensäure-Gemisch versehen ist und auf der Zellulosekörperstrecke vor dem Säure/Base-Farbindikator (4) ein Streckenstück (3) mit einer Puffersubstanz, vorzugsweise mit sekundärem Ammonium-Citrat, vorgesehen ist.

5. Indikator nach Anspruch 1, dadurch gekennzeichnet, dass im Bereich der Kontaktierungsöffnung (1a) für das Wasser am Zellulosekörper (1) eine zähflüssige Substanz, vorzugsweise ein Zucker, vorgesehen ist, um bei der Aktivierung des Indikators ein vorzeitiges Emporfliessen des Wassers zu verhindern.

6. Indikator nach Anspruch 1, dadurch gekennzeichnet, dass die Umhüllung (15, 16) eine Kunststoffausformung, vorzugsweise eine tiefgezogene Form aus transparentem PVC, ist.

7. Indikator nach Anspruch 1, dadurch gekennzeichnet, dass die Länge der Diffusionsstrecke in Diffusionsrichtung auf die maximale Haltbarkeit des zu überwachenden Gutes, vorzugsweise bei —20°C, abgestimmt ist.

8. Indikator nach Anspruch 2, dadurch gekennzeichnet, dass das Mischungsverhältnis von Natriumchlorid und Zitronensäure der Temperaturabhängigkeit der Haltbarkeit des zu überwachenden Gutes angepasst ist.

9. Verfahren zur Überwachung der Zeit/Temperaturgeschichte eines tiefgekühlten Gutes im Sinne eines Zeitintegrals seines Temperaturverlaufes, wobei der Diffusionsweg von Wasser auf einer Diffusionsstrecke erfasst wird, mit einem Indikator, nach einem oder mehreren der Ansprüche 1 bis 8, dadurch gekennzeichnet, dass man zur Aktivierung des Indikators den Zellulosekörper mit Wasser am Gut, sei dies der Feuchtigkeitsgehalt des Gutes selbst, sei dies ein zusätzlich vorgesehener Wasser- oder Eisvorrat, in Kontakt bringt.

10. Verfahren zur Überwachung der Zeit/Temperaturgeschichte eines tiefgekühlten Gutes, im Sinne eines Zeitintegrals seines Temperaturverlaufes, wobei der Diffusionsweg von Wasser auf einer Diffusionsstrecke erfasst wird, mit einem Indikator nach einem oder mehreren der Ansprüche 1 bis 8, dadurch gekennzeichnet, dass einerseits durch eine Diffusionsfront (2) entlang des Diffusionskörpers (1) die Abnahme der Haltbarkeit des zu überwachenden Gutes mit Hilfe einer auf eine bestimmte Temperatur, vorzugsweise auf —20°C, bezogenen Zeitskala (6) und andererseits, durch einen deutlichen Farbumschlag des Säure/Base-Indikators (4, 17) am Ende der Diffusionsstrecke, das Erreichen eines vorgegebenen Diffusionsgrenzwertes und damit das Verfallsdatum des Gutes angezeigt wird.

11. Verwendung eines Indikators zur Überwachung der Zeit/Temperaturgeschichte eines tiefgekühlten Gutes, im Sinne eines Zeitintegrals seines Temperaturverlaufes, wobei der Diffusionsweg von Wasser auf einer Diffusionsstrecke erfasst wird, nach einem oder mehreren der Ansprüche 1 bis 8, für die Überwachung des Qualitäts- bzw. Lagerzustandes von tiefgekühlten Lebensmitteln im Temperaturbereich von —25°C bis +5°C.

## Claims

1. Indicator to monitor the time/temperature history of a deep-frozen product in the sense of a time integral of its temperature development, whereby the diffusion path of water is detected on a diffusion section, consisting of an absorbant cellulose body (1, 18), serving as diffusion section, which is surrounded by a dampproof envelope (5, 15, 16), whereby the cellulose body is impregnated with a substance or a substance mixture and if required is furnished with an acid/base indicator and/or a time scale, characterized in that the envelope (5, 15, 16) has a contacting opening (1a, 21) for a water- or ice reserve and the cellulose body (1, 18) is provided with a water-soluble, hydrate-forming substance or substance mixture.

2. Indicator according to claim 1, characterized in that the water-soluble, hydrate-forming sub-stance is sodium chloride, cictric acid, a sugar, pre-ferably sodium chloride and/or citric acid or a mixture of two or more of these substances.

3. Indicator according to claim 1, characterized in that at the end of the cellulose body (1, 18) an acid/base indicator, preferably litmus paper (4, 17) is applied to indicate a predetermined threshold for the diffusion value and that the substance contains a solid acid or base.

4. Indicator according to claim 3, characterized in that the cellulose body (1, 18) is provided with a sodium chloride-citric acid mixture and on the cellulose body section in front of the acid/base colour indicator (4) a section piece (3) is provided with a buffer substance, preferably with secondary ammonium citrate.

5. Indicator according to claim 1, characterized in that in the region of the contacting opening (1a) for the water on the cellulose body (1) a viscous substance, preferably a sugar, is provided, in order to prevent a premature upward flow of the water on activation of the indicator.

6. Indicator according to claim 1, characterized in that the envelope (15, 16) is a synthetic material shaping preferably a deep-drawn form of transparent PVC.

7. Indicator according to claim 1, characterized in that the length of the diffusion section in the direction of diffusion is adjusted to the maximum durability of the material which is to be monitored, preferably at —20°C.

8. Indicator according to claim 2, characterized in that the mixture ratio of sodium chloride and citric acid is adapted to the temperature dependency of durability of the material which is to be monitored.

9. Process for the monitoring of the time/temperature history of a deep-frozen product in the sense of a time integral of its temperature development, whereby the diffusion path of water is detected on a diffusion section, with an indicator, according to one or more of claims 1 to 8, characterized in that to activate the indicator the cellulose body is brought into contact with water on the material, either with the moisture content of the material itself or with a water- or ice reserve which is additionally provided.

10. Process for the monitoring of the time/temperature history of a deep-frozen product, in the sense of a time integral of its temperature development, whereby the diffusion path of water is detected on a diffusion section, with an indicator according to one or more of claims 1 to 8, characterized in that, on one hand the decrease in durability of the material which is being monitored is indicated through a diffusion face (2) along the diffusion body (1) on a time scale (6) relating to a particular temperature, preferably to —20°C, and on the other hand, the reaching of a predetermined threshold value for the diffusion and hence the deterioration date of the material is indicated by a distinct colour change of the acid/base indicator (4, 17) at the end of the diffusion section.

11. Application of an indicator to monitor the time/temperature history of a deep-frozen product, in the sense of a time integral of its temperature development whereby the diffusion path of water is detected on a diffusion section, according to one or more of claims 1 to 8, for the monitoring of the quality- or storage condition of deep-frozen foodstuffs in the temperature range of —25°C to +5°C.

## Revendications

1. Indicateur pour la surveillance de l'évolution de la température en fonction du temps d'un produit surgelé, dans le sens d'une intégrale dans le temps de sa variation de température, où l'on détecte le trajet de diffusion d'eau sur un tronçon de diffusion, se composant d'un corps en cellulose (1, 18) absorbant et servant de tronçon de diffusion, qui est entouré d'une enveloppe (5, 15, 16) étanche à l'humidité, le corps en cellulose comprenant une substance ou un mélange de substances et présentant, le cas échéant, un indicateur acide/base et/ou une échelle des temps, caractérisé en ce que l'enveloppe (5, 15, 16) présente une ouverture de mise en contact (1a, 21) pour une réserve d'eau ou de glace et en ce que le corps en cellulose (1, 18) comprend une substance ou un mélange de substances soluble dans l'eau et formant un hydrate.

2. Indicateur selon la revendication 1, caractérisé en ce que la substance soluble dans l'eau et formant un hydrate est du chlorure de sodium, de l'acide citrique, un sucre, avantageusement du chlorure de sodium et/ou de l'acide citrique ou bien un mélange de deux ou plusieurs de ces substances.

3. Indicateur selon la revendication 1, caractérisé en ce qu'à l'extrémité du corps en cellulose (1, 18) est prévu un indicateur acide/base, avantageusement du papier de tournesol (4, 17) pour l'indication d'une valeur limite prédéterminée de diffusion et en ce que la substance contient un acide ou une base solide.

4. Indicateur selon la revendication 3, caractérisé en ce que le corps en cellulose (1, 18) comprend un mélange chlorure de sodium/acide citrique et en ce que sur le tronçon constitué par le corps en cellulose et avant l'indicateur coloré acide/base (4), est prévu un morceau de tronçon (3) avec une substance tampon, avantageusement du citrate d'ammonium secondaire.

5. Indicateur selon la revendication 1, caractérisé en ce que dans la zone de l'ouverture de mise en contact (1a) pour l'eau sur le corps en cellulose (1) est prévu une substance visqueuse, avantageusement un sucre, pour empêcher, par activation de l'indicateur, une remontée précoce de l'eau.

6. Indicateur selon la revendication 1, caractérisé en ce que l'enveloppe (15, 16) est un élément formé, en matière synthétique, avantageusement une forme emboutie en PVC transparent.

7. Indicateur selon la revendication 1, caractérisé en ce que la longueur du tronçon de diffusion dans la direction de diffusion, est déterminée à partir de la conservabilité maximum de la marchandise à surveiller, avantageusement à —20°C.

8. Indicateur selon la revendication 2, caractérisé en ce que le rapport de mélange du chlorure de sodium et de l'acide citrique est adapté à la dépendance de la conservabilité de la marchandise à surveiller avec la température.

9. Procédé de surveillance de l'évolution de la température en fonction du temps d'un produit surgelé, dans le sens d'une intégrale dans le temps de sa variation de température, où le parcours de diffusion d'eau est détecté sur un tronçon de diffusion, avec un indicateur, selon l'une quelconque des revendications 1 à 8, caractérisé en ce que, pour activer l'indicateur, on amène le corps en cellulose en contact avec de l'eau sur la marchandise, que ce soit la teneur en humidité de la marchandise elle-même, ou que ce soit une réserve d'eau ou de glace prévue en supplément.

10. Procédé de surveillance de l'évolution de la température en fonction du temps d'un produit surgelé, dans le sens d'une intégrale dans le temps de sa variation de température, où le parcours de diffusion de l'eau est détecté sur un tronçon de diffusion, avec un indicateur selon l'une quelconque des revendications 1 à 8, caractérisé en ce que l'on décèle, d'une part, par un front de diffusion (2) le long du corps de diffusion (1) la diminution de la conservabilité de la marchandise à surveiller à l'aide d'une échelle des temps (6) rapportée à une température déterminée, avantageusement à —20°C et d'autre part, par un virage net de la couleur de l'indicateur acide/base (4, 17) à la fin du tronçon de diffusion, l'atteinte d'une valeur limite prédéterminée de diffusion et ainsi la date à laquelle le produit est périmé.

11. Utilisation d'un indicateur pour la surveillance de l'évolution de la température en fonction du temps d'un produit surgelé, dans le sens d'une intégrale dans le temps de sa variation de température, où le parcours de diffusion d'eau sur un tronçon de diffusion est détecté, selon l'une quelconque des revendications 1 à 8, pour la surveillance de la qualité ou de l'état de conservation de produits alimentaires surgelés dans une plage de température de —25°C à +5°C.

FIG.1

FIG.2

FIG.3

0 031 826

FIG. 4

FIG. 5

FIG. 6

a)    FIG.7    b)

11